# EUROPÄISCHE PATENTSCHRIFT

(11) **EP 1 987 130 B1**
(45) Veröffentlichungstag und Bekanntmachung des Hinweises auf die Patenterteilung: **05.08.2009**
(21) Anmeldenummer: 06829219.2
(22) Anmeldetag: 30.11.2006
(51) Int. Cl.: C12M 1/113

(54) **FERMENTATIONSEINRICHTUNG UND VERFAHREN ZUR GEWINNUNG VON BIOGAS**
FERMENTATION DEVICE AND METHOD FOR RECOVERING BIOGAS
DISPOSITIF DE FERMENTATION ET PROCEDE DE RECUPERATION DE BIOGAZ

(30) Priorität: 05.12.2005 DE 102005057979
(43) Veröffentlichungstag der Anmeldung: 05.11.2008
(73) Patentinhaber: STRABAG Umweltanlagen GmbH, 01067 Dresden (DE)
(72) Erfinder: BÜCHNER, Thomas, 01731 Kreischa (DE); LANGHANS, Gerhard, 01159 Dresden (DE)
(86) Internationale Anmeldenummer: PCT/EP2006/011524
(87) Internationale Veröffentlichungsnummer: WO 2007/065598

(56) Entgegenhaltungen:
- EP-A2- 0 617 120
- DE-A1- 3 239 304
- US-A- 5 773 526

## Beschreibung

Die Erfindung betrifft eine Fermentationseinrichtung zum biologischen Abbau von organisches Material enthaltendem Substrat und zur Gewinnung des beim Abbau entstehenden Biogases mit einem längserstreckten geschlossenen Behälter mit einer Einbringöffnung für das Substrat und Austragsöffnungen für die Abbauprodukte, wobei der Behälter mehrere Reaktionsvolumenzellen aufweist, die jeweils mindestens eine individuell angetriebene Rühreinrichtung zum Durchmischen des Substrats enthalten, sowie ein Verfahren zum Betreiben der Fermentationseinrichtung.

Bei der Fermentation von organisches Material enthaltendem Substrat, z.B. von Bioabfällen, Rückständen aus der Nahrungsmittelproduktion oder nachwachsenden Rohstoffen, unterscheidet man zwischen der sog. Naßvergärung und der sog. Trockenvergärung. Während bei der Nassvergärung eine fließfähige Suspension aus dem Substrat hergestellt wird, die in einem z.B. als Schlaufenreaktor mit innenliegender Schlaufenströmung ausgebildeten Biogasreaktor behandelt wird, wird das Substrat bei der Trockenvergärung z.B. an einem Ende in einen liegenden geschlossenen Behälter eingebracht, darin mittels einer Rühreinrichtung unter Bildung von Biogas umgewälzt und das behandelte Substrat am anderen Ende aus dem Behälter abgezogen. Die Verfahren der Trockenvergärung werden insbesondere dann als Alternative zu denen der Nassvergärung eingesetzt, wenn das zu behandelnde Substrat sehr feststoffreich ist und deshalb die notwendige Verdünnung bis in den Betriebsbereich der Nassvergärung bei Feststoffkonzentrationen von unter ca. 15% unwirtschaftlich ist, bzw. wenn die notwendige aber kostenaufwendige Störstoffentfrachtung bei der Nassvergärung aus sonstigen prozesstechnischen Gründen nicht erforderlich ist. Trockenvergärungen werden bevorzugt bei Feststoffgehalten bis ca. 35% im Fermentereintritt gefahren und können als stehende oder liegende Reaktoren ausgebildet sein. In der Regel werden sie als Pfropfenstromreaktoren betrieben, wobei das zudosierte Substrat am Fermenteranfang nur wenig verdünnt oder völlig unverdünnt in seiner Eingangskonzentration vorliegt. Der vorhandene Anteil an biologisch abbaubarer Organik wird über die Reaktorlänge bis zur Entnahme durch eine mikrobielle Biozynose anaerob-biochemisch bevorzugt in Biogas umgesetzt.

Eine spezielle Ausführungsform des Pfropfenstromreaktors ist in der EP 0 961 762 A1 und der EP 0 617 120 B1 beschrieben. Bei diesem Reaktor ist der Pfropfenstrom in hydraulisch verbundene, nacheinander geschaltete Reaktionsvolumenzellen mit separaten Rührwerken aufgeteilt, wobei die Rührwerke separat ansteuerbar sind und damit innerhalb des Pfropfenstromzonen individuell optimierbarer Stoffumsatzleistung geschaffen werden. Das Dokument DE-A-3239304 beschreibt einen Reaktor zur Erzeugung von Biogas aus Gülle. Die Durchmischung und Bewegung der Gülle wird mittels eines individuell angetriebenen Rührwerkes erreicht. Die Rührwerke weisen Rührflügel auf, die einander überlappen Können.

Die aus der Nassvergärung im volldurchmischten Reaktor definierten Prozess-parameter, wie mittlere hydraulische Verweilzeit und organische Raumbelastung werden auch für die Charakterisierung des Pfropfenstrom-Trockenfermenters übernommen. Dabei ist ihre verfahrenstechnische Gültigkeit jedoch begrenzt, da ihre Definition darauf beruht, Eingangsparameter auf den gesamten Reaktionsraum zu beziehen. Beim Pfropfenstromfermenter der Trockenvergärung wird das in den Reaktor gegebene Substrat jedoch nicht sofort auf das gesamte Reaktionsvolumen verdünnt, sondern liegt im Eingangsbereich des Reaktionsraumes unverdünnt in seiner Anfangskonzentration vor. Über den Transportweg durch den Reaktor laufen die biochemischen Prozesse ab mit entsprechend fortschreitender qualitativer Veränderung des Eingangssubstrats. Dabei handelt es sich im Wesentlichen um einen Abbau der organischen Stoffe. Nach der definierten mittleren Verweilzeit verlässt das über die Reaktorlänge abgebaute Endprodukt schließlich den Reaktionsraum.

Trockenfermenter wurden hauptsächlich zur Behandlung von feststoffreichen Bioabfällen und Siebfraktionen aus Restmüll entwickelt. Bei diesen Stoffströmen bestehen zwischen 1/3 und 2/3 der eingetragenen Trockensubstanz aus organischem Material, definiert durch den Glühverlust des Feststoffes. Damit wird die zugeführte organische (Glühverlust)- Fracht bezogen auf den Gärraum, zur Definition der organischen Raumbelastung des Trockenfermenters verwendet. Mikrobiologisch relevant ist jedoch nur der bakteriell verwertbare Anteil des Glühverlustes. Nicht oder nur sehr schwer abbaubare Komponenten wie Kunststoff, lignifiziertes Holz und Fasern, aber auch mehr oder weniger durch Lignine oder Kieselsäureverbindungen geschützte Zellulosebestandteile bedeuten für die anaeroben Bakterien keine tatsächliche organische Belastung, da sie nicht oder nur sehr langsam hydrolysiert werden und damit nicht milieu- und populationsrelevant Einfluss auf den anaeroben Metabolismus nehmen. Entsprechend liegen die Trockensubstanzabbaugrade zwischen Fermentereintritt und -austritt bei 40 bis 60%, gemessen als Veränderung des Glühverlustes in der Probe.

Andere Verhältnisse sind bei der Vergärung von z.B. organikreichen Abprodukten der Nahrungsgüterindustrie und bei Einsatz nachwachsender Rohstoffe als Substrat gegeben. Hier liegt der Anteil organischer Substanz an der gesamten Trockenmasse in der Regel zwischen 85 und 98%. Abbaugrade dieser Organik werden zwischen 75 und 90% erwartet. Somit kommt eine hohe biologische Relevanz dieser dem Fermenter zugeführten Organikfracht zum Ausdruck.

Es ist auch schon ein liegender Propfenstromreaktor mit längsangeordneter zentraler Rührerwelle vorgeschlagen worden, bei dem an radialen Stegen befestigte Rührbalken an der zylindrischen Reaktorinnenwand entlang geführt werden. Alternativ ist eine Gaseinpressung über Bodendüsen möglich, die durch ihre Schwallbildung, Mischung und Schwimmdeckenzerstörung bewirken soll. Dabei ist auch vorgesehen, über die Reaktorlänge an verschiedenen Stellen Substrat zu dosieren, ohne verfahrenstechnisch definierte Verteilung der Dosierpunkte. Dieses beschriebene Vergärungsverfahren hat beim Einsatz der oben spezifizierten Gärsubstrate mit hoher OTS-Konzentration und hohem erreichbaren Abbau eine Reihe verfahrenstechnischer und hydraulischer Nachteile. Über die Länge des Pfropfenstromreaktors entsteht durch den hohen Abbau an suspendierter Organik ein großes Feststoff- Konzentrationsgefälle zwischen den Eingangstrockensubstanzkonzentrationen von über 30% und den Ablaufwerten von unter 10%. Ohne hydraulisch genau definierte Strömungsführung im Reaktor besteht deshalb unter diesen substratspezifischen Bedingungen das Problem eines unkontrollierten Konzentrationsausgleichs über die Reaktorlänge. Feststoffe aus dem Eingangsbereich werden distributiert über die Reaktorlänge und damit ungenügend abgebaut aus dem Reaktor wieder entnommen, da die Pfropfenstromcharakteristik durch Längs- und Quervermischungseffekte in dem durch Abbau zunehmend wässriger werdenden Gärmedium überlagert wird (sinkende Viskosität). Unterstützt wird dieser negative Effekt durch die beschriebene Substrateindüsung und Begasung im Reaktionsraum infolge der dreidimensionalen Mediendissipation. Mit zunehmend verringerter Medienviskosität neigt frischdosiertes Material außerdem dazu, sich nach dem Dichteunterschied in Schwimm- und Sinkschichten zu separieren und infolge des gehemmten Stofftransports im Bereich der weitgehend ruhenden Partikelansammlungen einem verzögerten mikrobiellen Abbau zu unterliegen.

Der vorliegenden Erfindung liegt die Aufgabe zugrunde, eine Fermentationseinrichtung sowie ein Verfahren zum Betrieb der Fermentationseinrichtung der eingangs
genannten Art so auszugestalten, dass eine Überlastung der anaeroben Biozönose im Eingangsbereich der Fermentationseinrichtung vermieden und die Prozessstabilität als Pfropfenstrom bei der Vergärung von Substraten mit leicht abbaubaren hohen Trockensubstanzgehalten gewährleistet wird.

Diese Aufgabe wird erfindungsgemäß bei der Fermentationseinrichtung dadurch gelöst, dass in Längserstreckung des Behälters verteilt mindestens zwei Eintragsöffnungen für das Substrat vorgesehen sind, wobei die Eintragsöffnungen im Überlappungs- oder Tangierbereich benachbarter Rühreinrichtungen angeordnet sind.

Aufbauend auf dem apparativen Grundkonzept des Trockenfermenters gemäß der EP 0 617 120 B1 mit separat ansteuerbaren, quer liegenden Rührwerkseinheiten wurde eine Verfahrensführung einschließlich zugehöriger Vorrichtungselemente entwickelt, die eine effiziente anaerobe Verwertung der oben genannten energiereichen Organikfraktionen ermöglichen.

Bei Untersuchungen an ausgeführten Pfropfenstromreaktoren nach dem patentierten Rührzellenprinzip mit mehreren Reaktionsvolumenzellen mit individuellen Rühreinrichtungen (Rührzellen) ist für die Vergärung von Bioabfall und Restmüllfraktionen überraschenderweise festgestellt worden, dass 60 bis 70% der über den gesamten Reaktor umgesetzten Organik schon im Bereich der ersten und zweiten Rührzelle des Reaktionsraumes hydrolysiert und auch schon simultan methanisiert werden. Im restlichen Reaktionsraum erfolgt dann nur ein sehr langsamer Abbau der verbliebenen, bakteriell nur noch schwer verwertbaren Organikbestandteile. Für konventionelle Bioabfälle ist diese Prozesscharakteristik gewollt, um einen möglichst hohen Umsatz auch bei abbauresistenten organischen Verbindungen zu erreichen. Unterstützt wird dieses Anliegen durch die Betriebscharakteristik des Pfropfenstromprinzips.

Der Erfindung liegt die Erkenntnis zugrunde, dass es für die leichtabbaubaren Inhaltsstoffe bei Nahrungsgüterrückständen und nachwachsenden Rohstoffen (überwiegend Stärke, Zucker, Eiweiße) erforderlich ist, den weitgehenden Umsatz im Eingangsbereich des Fermenters durch geeignete Maßnahmen zu reduzieren und statt dessen ein annähernd ausgeglichenes Verwertungsprofil über den gesamten Reaktionsraum zu erreichen. Dabei darf die Pfropfenströmung jedoch nicht gestört werden und frisches Material ist vom Austritt fernzuhalten. Dazu muss der Reaktionsraum auch über den gesamten Reaktorquerschnitt gleichmäßig für die biologische Aktivität genutzt werden und Schwimm- und Sinkschichtenbildung ist zu minimieren. Damit werden sowohl Millieustörungen bei der Biozönose als auch prozessbeeinträchtigende Folgereaktionen wie starke Schaumbildung mit Flotationserscheinungen weitgehend vermieden.

Der wesentliche Erfindungsgedanke besteht darin, statt der herkömmlichen intervallweisen Substratdosierung ausschließlich über die Stirnseite des Gärreaktors die einzutragende Substratmenge an mehreren Eintragspunkten über der Reaktorlänge verteilt zu dosieren. Hierzu wird vorzugsweise die einzutragende Substratmenge aufgeteilt und portionsweise außer an der Stirnseite auch an weiteren Eintragspunkten in den Reaktor eingebracht. Zu diesem Zweck stehen die Eintragsöffnungen über Leitungen mit einer gemeinsamen Zuführung für das Substrat in Verbindung.

Vorzugsweise ist jeder Eintragsöffnung eine eigene Dosiereinrichtung zugeordnet, so dass ein ausgeglichenes Verwertungsprofil über die gesamte Reaktorlänge eingestellt werden kann. Zur Steuerung der Dosierung stehen die Dosiereinrichtungen zweckmäßigerweise mit einer zentralen Steuereinheit in Wirkverbindung.

Gemäß einer Weiterbildung des Erfindungsgedankens sind im Überlappungs- oder Tangierbereich der Rühreinrichtung strömungsbrechende Elemente quer zur Längserstreckung des Behälters vorgesehen. Dadurch wird ein Abtreiben von Flotatanreicherungen an der Flüssigkeitsoberfläche zum Fermenterende verhindert. Zu solchen Flotatanreicherungen kann es in Abhängigkeit der Konsistenz des dosierten Substrats im Bereich der Substratdosierung durch lokales Aufrahmen des frischen Dosierguts kommen. Die strömungsbrechenden Elemente gewährleisten ein Einmischen und Verarbeiten dieser Flotatanreicherungen in der jeweiligen Reaktionsvolumenzelle.

Die Erfindung betrifft ferner ein Verfahren zum biologischen Abbau von organisches Material enthaltendem Substrat und zur Gewinnung des beim Abbau entstehenden Biogases, wobei das Substrat in einen längserstreckten geschlossenen Behälter eingebracht wird, im Behälter mehrere Reaktionszonen durchströmt, in denen es mittels jeweils individuell angetriebener Rühreinrichtungen umgewälzt wird und Abbauprodukte aus dem Behälter abgezogen werden.

Verfahrensseitig wird die gestellte Aufgabe dadurch gelöst, dass das Substrat an mindestens zwei über die Längserstreckung des Behälters verteilten Stellen jeweils im Überlappungs- oder Tangierbereich benachbarter Rühreinrichtungen in den Behälter eingebracht wird.

Bevorzugt wird das Substrat im Gegensatz zur herkömmlichen intervallweisen Dosierung ausschließlich über die Stirnseite des Gärreaktors aufgeteilt und portionsweise außer an der Stirnseite auch an weiteren diskreten Eintragsstellen über der Reaktorlänge verteilt zudosiert. Diese Dosierungen können zyklisch geschaltet nacheinander erfolgen, aber auch gleichzeitig, je nach den substratabhängigen Optimierungsergebnissen im Anlagenbetrieb.

Vorzugsweise wird die Dosierung an den Eintragstellen so gesteuert, dass über das gesamte Behältervolumen ein ausgeglichenes Verwertungsprofil erreicht wird.

Die Erfindung eignet sich insbesondere für Fermentationseinrichtungen, die für eine Biogasgewinnung durch anaeroben Abbau von leichtabbaubare Inhaltsstoffe enthaltenden Nahrungsgüterrückständen oder nachwachsenden Rohstoffen vorgesehen sind.

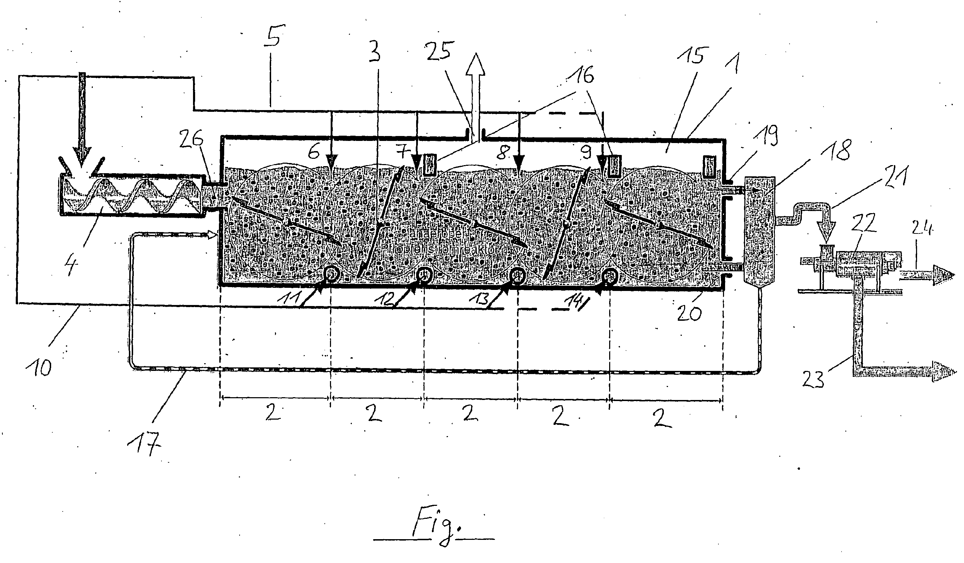

Im Folgenden soll die Erfindung anhand eines in der Figur schematisch dargestellten Ausführungsbeispiels näher erläutert werden:

Die Figur zeigt einen Pfropfenstromfermenter mit mehreren Reaktionsvolumenzellen, die jeweils individuell angetriebene Rühreinrichtungen enthalten.

Das vorliegende Ausführungsbeispiel betrifft einen liegenden Pfropfenstromfermenter zur Erzeugung von Biogas, welches über eine Abzugsöffnung 25 abgeführt wird. Der Fermenter weist einen länglichen Behälter 1 mit mehreren in Reihe geschalteten Reaktionsvolumenzellen (Rührzellen) 2 auf, die jeweils mit langsam laufenden Balkenrührwerken 3 an Achsen senkrecht zur Durchströmungsrichtung des Fermenters ausgestattet sind. Jedes von einem Rührwerk 3 beaufschlagte Volumenelement bildet eine Reaktionsvolumenzelle 2 mit annähernd gleichen Prozessbedingungen. Durch konstruktive Wahl der Rührwerksabstände werden die Rührbalken benachbarter Rührwerke 3 etwa tangierend oder mit einer prozesstechnisch relevanten Überdeckung vorgesehen, wobei eine Anpassung an die am Anlagenstandort erwarteten Eigenschaften des Gärmediums erfolgt. Eine Längsströmung im Fermenter induzieren die Rührwerke 3 nicht. Diese entsteht bevorzugt durch das Zusammenwirken von Substratdosierung und Faulgutentnahme in Form des schon beschriebenen Pfropfenstromes. Da andererseits der hauptsächliche Organikabfall bei der Bioabfallvergärung etwa im Volumenbereich des ersten und zweiten Rührwerkes 3 beobachtet wird, sind diese Reaktionsvolumenzellen 3 geeignete Kenngrößen für die Festlegung der Dosierverteilung längs des Fermenters. Neben der Stimseitendosierung über eine Schneckenfördereinrichtung 4 und eine Einbringöffnung 26 vor dem ersten Rührwerk 3 erfolgen Nachdosierungen weiterer Substratzugaben jeweils in der Tangier- bzw. Kämmzone zwischen zwei benachbarten Rührwerken 3. Die Nachdosierungen können über eine Leitung 5 und Dosiereinrichtungen 6, 7, 8 und 9 über Kopf erfolgen oder alternativ als bodennahe Dosierung über Leitung 10 und Dosiereinrichtung 11, 12, 13 und 14. Dabei kann es sich substratsspezifisch als vorteilhaft erweisen, nicht in jedem Fall nach jedem Rührwerk 3, sondern gegebenenfalls erst nach jedem zweiten einen Substratstrom zu dosieren. Diese Festlegung hängt von dem jeweils sich ausbildenden Abbauprofil der Organik im Fermenter ab.

Im Entnahmebereich nach der letzten Reaktionsvolumenzelle 2 wird nicht mehr dosiert, wobei eine Dosierung zwischen den letzten beiden Reaktionsvolumenzellen auch nur unter speziellen Prozessbedingungen erfolgt. In der Regel werden die beiden letzten Reaktionsvolumenzellen zum Substratabbau auf die gewünschte Entnahmekonzentration genutzt. Außerdem wird durch ein spezielles Zusammenwirken von Rühr- und Ruhezeiten in diesem Fermenterbereich eine Entkopplung von Feststoff- und Flüssigkeitsverweilzeit erreicht, wodurch besonders hohe Organikumsatzraten ermöglicht werden.

Entscheidendes Wirkprinzip der Aufteilung der Substratdosierung ist die Reduzierung der auf die Bakterien wirkenden lokalen organischen Raumbelastung. Während die auf den gesamten Reaktionsraum gerechnete mittlere Raumbelastung unverändert bleibt, zeigen sich drastisch reduzierte lokale Belastungswerte. Bei ausschließlicher Dosierung an der Reaktorstimseite erfolgt der Hauptumsatz überraschenderweise im Bereich der ersten Reaktionsvolumenzelle. Hier wirkt demnach voll die Raumbelastung aus der organischen Substratfracht, bezogen auf das diskrete Rührvolumenelement. Für einen Reaktortyp mit z.B. fünf Rührwerken bedeutet das eine fünffach höhere tatsächliche Raumbelastung in dem relevanten Bereich im Vergleich zu der über das gesamte Reaktorvolumen gemittelten.

Verteilt man gemäß der Erfindung die Substratdosierung auf verschiedene Reaktionsvolumenzellen, so verringert sich die lokale Raumbelastung und nähert sich der über das Gesamtvolumen gemittelten an. Damit wird die Biozönose stark entlastet und die Prozessstabilität bei der Vergärung organikreicher Substrate wesentlich verbessert.

Die erforderliche Steuerung der Substratverteilung in der jeweiligen Reaktionsvolumenzelle sowie die Minimierung eines Konzentrationsausgleichs über die Reaktorlänge aufgrund des abbaubedingten Feststoffkonzentrationsgefälles wird durch die Substratzuführungen an den genau definierten Eintragspunkten 6, 7, 8 und 9 beziehungsweise 11, 12, 13 und 14 in Verbindung mit einer auf die Dosierung abgestimmten Steuerung der Rührwerke 3 erreicht. Dazu arbeiten die dem Dosiereintritt benachbarten Rührwerke 3 mit entgegengesetzten Drehrichtungen, um entweder Dosiergut von oben in ihren Kämmbereich einzutragen oder Bodenmaterial anzuheben. Die Hauptdrehrichtung außerhalb der Dosierintervalle wird in Abhängigkeit der dominanten organikhaltigen Sediment- oder Flotatausbildung so gewählt, dass die jeweilige Fraktion vom Reaktorende wegbewegt und angehoben beziehungsweise untergemischt wird. Ungewollte Längsströmungen, wie sie durch den bekannten Golfstrom-Effekt bei Flüssigkeitsfreistrahlen oder Gaseinpressung in den Reaktionsraum eintreten, werden durch die beschriebene Kombination von Verfahrenführung und Vorrichtung stark reduziert.

In Abhängigkeit der Konsistenz und Benetzbarkeit der dosierten Substrate kann es im Bereich der Substratdosierungen 6, 7, 8 und 9 über den Gasraum 15 zu einem lokalen Aufrahmen des frischen Inputs kommen. Um ein Abtreiben dieser Flotatanreicherungen an der Flüssigkeitsoberfläche zum Fermenterende zu verhindern und das Einmischen und Verarbeiten in der jeweiligen Reaktionsvolumenzelle zu gewährleisten, ist die Einbringung von strömungsbrechenden Elementen 16 quer zur Fließrichtung im Kämmbereich der Rührwerke 3 vorgesehen.

In Ergänzung zur beschriebenen Dosieraufteilung ist auch eine gezielte hydraulische Beeinflussung des Pfropfenstromprofils im Reaktor wünschenswert. Dazu wird bedarfsweise Gärmedium vom Ende des Reaktors über eine Rücklaufleitung 17 zum Eingang zurückgeführt. In dieser Weise ist die Längsverteilung des Konzentrationsprofils im Reaktor beeinflussbar. Bedarfsweise lässt sich zur Beherrschung spezieller Prozesszustände eine Dosiercharge durch erhöhten Durchfluss auf zwei oder mehr Reaktionsvolumenzellen 2 verteilen und das Konzentrationsprofil auseinander ziehen, was zu einer weiteren lokalen Konzentrationsminderung führt. Über die Rücklaufleitung 17 erfolgt darüber hinaus eine Rückbeimpfung des Fermentereingangsbereichs mit zusätzlichen Anaerob-Bakterien, wodurch die biomassebezogene Schlammbelastung reduziert ist. Zudem wird in Bereichen des Reaktors, wo durch starke hydrolytische Produktion von kurzkettigen organischen Säuren als Stoffwechselzwischenprodukte ein hemmender Abfall des pH-Wertes auftreten kann, durch den alkalisch gepufferten Fermenterablauf eine pH-Stabilisierung erreicht. Bei hohen Rückführraten nähert sich das hydraulische Gesamtsystem Fermenter/Rückführung als Grenzfall der Charakteristik eines volldurchmischten Reaktors mit dem Fermentervolumen als Strömungsrohr. Diese Flexibilität in der Prozesscharakteristik ermöglicht eine optimierte verfahrenstechnische Anpassung des Systems an die jeweiligen Betriebsbedingungen. Begrenzt wird die einstellbare Rückführrate über die Rückführleitung 17 durch die zunehmende Substratkonzentration am Fermenterauslauf bei Übergang von der Pfropfenströmungszur Rührkesselcharakteristik mit der daraus resultierenden Umsatzminderung durch Ausschleusung noch nicht metabolisierter organischer Verbindungen.

Durch einen speziell gestalteten Fermenteraustrag 18 wird dieser Effekt minimiert, indem noch nicht hydrolisierte partikuläre Feststoffe vom Austrag ferngehalten werden, was zu der beschriebenen Rückkopplung der Feststoff- und Flüssigkeitsverweilzeiten im Reaktionsraum führt. Dazu werden am Reaktorende installierte Entnahmestutzen 19 und 20 genutzt, die in verschiedenen Höhen am Reaktorende vorgesehen sind. Über den Entnahmestutzen 19 wird feststoffarme Flüssigkeit aus dem Behälter 1 abgezogen und in einen Sammelbehälter 18 eingeleitet. Die mit Trockensubstanz und Sedimenten angereicherte Bodenfraktion wird über den Entnahmestutzen 20 in den unteren Teil des Sammelbehälters 18 eingebracht. Über eine Leitung 21 wird das Gärgut entnommen und einer Entwässerungseinrichtung 22 zugeführt. Dabei entsteht Presswasser, das zum Beispiel als Flüssigdünger über Leitung 23 abgeführt wird. Abgeschiedene Gärreststoffe werden über Leitung 24 abgezogen. Am unteren Ende des Sammelbehälters 18 wird Faulgut und Presswasser abgeführt und über die Rücklaufleitung 17 zum Eingangbereich des Reaktors zurückgeführt.

Entstehen langzeitlich signifikante Mengen inerter Bodensedimente, so lassen sich diese durch Drehrichtung des letzten Rührwerks 3 entgegen dem Uhrzeigersinn in den Bereich des bodennahen Entnahmestutzens 20 verlagern und gezielt aus dem Reaktor entnehmen.

Erfolgt die Gärgutentnahme am Ende einer Stillstandsphase des letzten Rührwerks 3, sedimentieren die verbliebenen unabgebauten Feststoffe in dem wässriger gewordenen Gärmedium, so dass sich über die Flüssigkeitshöhe ein Konzentrationsprofil ausbildet mit einer Verarmung an suspendierten Partikeln in Richtung Flüssigkeitsoberfläche. Wird jetzt Gärmedium über den im oberen Drittel der Fermenterhöhe angeordneten Entnahmestutzen entnommen, handelt es sich dabei um Medium mit der relativ zur sonstigen Konzentration geringeren partikulären Feststoffkonzentration. Nach dem jeweiligen Entnahmezyklus wird mittels Rührwerksbewegung das suspendierte Material wieder aufgelockert und im Volumen verteilt, um die Stoffübergangsbedingungen für die weitere Hydrolyse zu intensivieren.

Im Bodenbereich werden Sedimente innerhalb der Reaktionsvolumenzellen durch die Rührwerksbalken weiter transportiert. Begrenzte lokale Sedimentanreicherungen können in der bodennahen Kämmzone der Rührwerke 3 auftreten. Dieser Effekt lässt sich verringern, indem neben der Überkopfdosierung über Leitung 5 eine Dosierung insbesondere pumpfähig angemischter Substratchargen in Bodennähe über Leitung 10 in die Kämmzonen der Rührwerke 3 erfolgt. Dabei werden Sedimente durch eine aufgrund des eingetragenen Substrats erfolgten Freistrahlströmung aufgewirbelt und in Längsrichtung wieder in den Wirkbereich der Rührwerksbalken transportiert. Gleichzeitig kann die Einmischung und Verteilung des frischen Substrats in der Reaktionsvolumenzelle verbessert werden.

## Patentansprüche

1. Fermentationseinrichtung zum biologischen Abbau von organisches Material enthaltendem Substrat und zur Gewinnung des beim Abbau entstehenden Biogases mit einem längserstreckten geschlossenen Behälter (1) mit einer Einbringöffnung (26) für das Substrat und Austragsöffnungen (19, 20) für die Abbauprodukte, wobei der Behälter (1) mehrere Reaktionsvolumenzellen (2) aufweist, die jeweils mindestens eine Rühreinrichtung (3) zum Durchmischen des Substrats enthalten, **dadurch gekennzeichnet, dass** in Längserstreckung des Behälters (1) verteilt mindestens zwei Eintragsöffnungen (6, 7, 8, 9, 11, 12, 13, 14) für das Substrat vorgesehen sind, wobei die Eintragsöffnungen ((6, 7, 8, 9, 11, 12, 13, 14) im Überlappungs- oder Tangierbereich benachbarter Rühreinrichtungen angeordnet sind.

2. Fermentationseinrichtung nach Anspruch 1, **dadurch gekennzeichnet, dass** die Eintragsöffnungen (6, 7, 8, 9, 11, 12, 13, 14) über Leitungen (5, 10) mit einer gemeinsamen Zuführung für das Substrat in Verbindung stehen.

3. Fermentationseinrichtung nach Anspruch 1 oder 2, **dadurch gekennzeichnet, dass** jeder Eintragsöffnung (6, 7, 8, 9, 11, 12, 13, 14) eine eigene Dosiereinrichtung zugeordnet ist.

4. Fermentationseinrichtung nach Anspruch 3, **dadurch gekennzeichnet, dass** die Dosiereinrichtungen mit einer zentralen Steuereinheit in Wirkverbindung stehen.

5. Fermentationseinrichtung nach einem der Ansprüche 1 bis 4, **dadurch gekennzeichnet, dass** im Übertragungs- oder Tangierbereich der Rühreinrichtungen (3) strömungsbrechende Elemente (16) quer zur Längserstreckung des Behälters (1) vorgesehen sind.

6. Verfahren zum biologischen Abbau von organisches Material enthaltendem Substrat und zur Gewinnung des beim Abbau entstehenden Biogases, wobei das Substrat in einen längserstreckten geschlossenen Behälter (1) eingebracht wird, im Behälter (1) mehrere Reaktionszonen (2) durchströmt, in denen es mittels separater Rühreinrichtungen (3) umgewälzt wird und Abbauprodukte aus dem Behälter (1) abgezogen werden, **dadurch gekennzeichnet, dass** das Substrat an mindestens zwei über die Längserstreckung des Behälters verteilten Stellen (6, 7, 8, 9, 11, 12, 13, 14) jeweils im Überlappungs- oder Tangierbereich benachbarter Rühreinrichtungen in den Behälter (1) eingebracht wird.

7. Verfahren nach Anspruch 6, **dadurch gekennzeichnet, dass** die in den Behälter (1) einzutragende Substratmenge aufgeteilt wird und außer an der Stirnseite des Behälters (1) auch an weiteren diskreten Eintragsstellen (6, 7, 8, 9, 11, 12, 13, 14) über die Behälterlänge verteilt in den Behälter (1) dosiert wird.

8. Verfahren nach Anspruch 7, **dadurch gekennzeichnet, dass** die Dosierungen zyklisch oder nacheinander geschaltet werden.

9. Verfahren nach Anspruch 7 oder 8, **dadurch gekennzeichnet, dass** die Dosierungen an den Eintragsstellen (6, 7, 8, 9, 11, 12, 13, 14) so gesteuert werden, dass über das gesamte Behältervolumen ein ausgeglichenes Verwertungsprofil erreicht wird.

## Claims

1. A fermentation device for the biological degradation of a substrate containing organic material and for the recovery of the biogas produced in the degradation process comprising an elongated sealed container (1) with a feed opening (26) for the substrate and removal openings (19, 20) for the degraded products, wherein the container (1) is equipped with several reaction volume cells (2) each of which has at least one mixing unit (3) for mixing the substrate, **characterized in that** at least two feed points (6, 7, 8, 9, 11, 12, 13, 14), spaced over the length of the container (1), are provided for the substrate, wherein the feed points (6, 7, 8, 9, 11, 12, 13, 14) are arranged in the overlap or tangential region of adjacent mixing units.

2. A fermentation device according to claim 1, **characterized in that** the feed points (6, 7, 8, 9, 11, 12, 13, 14) are connected via conduits (5, 10) to a common intake for the substrate.

3. A fermentation device according to claim 1 or 2, **characterized in that** each feed point (6, 7, 8, 9, 11, 12, 13, 14) is assigned its own metering unit.

4. A fermentation device according to claim 3, **characterized in that** the metering units are in operative connection with a central control unit.

5. A fermentation device according to one of claims 1 to 4, **characterized in that** flow-disrupting elements (16) are provided in the transfer or tangential region of the mixing units (3) transverse to the length of the container (1).

6. A method for the biological degradation of a substrate containing organic material and for the recovery of the biogas produced in the degradation process, wherein the substrate is fed into an elongated sealed container (1), flows inside the container (1) through several reaction zones (2) in which it is made to circulate by means of separate mixing units (3), and degraded products are removed from the container (1), **characterized in that** the substrate is fed into the container (1) at at least two points (6, 7, 8, 9, 11, 12, 13, 14) spaced over the length of the container, in each case in the overlap or tangential region of adjacent mixing units.

7. A method according to claim 6, **characterized in that** the amount of substrate that is to be fed into the container (1) is divided up and metered into the container (1) not only at the front end of the container (1) but also at other discrete feed points (6, 7, 8, 9, 11, 12, 13, 14) spaced over the length of the container.

8. A method according to claim 7, **characterized in that** the metering is switched cyclically or successively.

9. A method according to claim 7 or 8, **characterized in that** the metering at the feed points (6, 7, 8, 9, 11, 12, 13, 14) is controlled in such a way that a balanced utilization profile is achieved across the entire container volume.

## Revendications

1. Dispositif de fermentation pour la décomposition biologique de substrat contenant de la matière organique et de récupération de biogaz se dégageant lors de la décomposition avec un conteneur fermé et allongé (1) avec un orifice de chargement (26) pour le substrat et des orifices d'évacuation (19, 20) des produits de la décomposition, tout en notant que le conteneur (1) présente plusieurs cellules à volume de réaction (2), qui comportent respectivement au moins un dispositif de mélange (3) pour mélanger le substrat, **caractérisé en ce que** dans l'allongement longitudinal du conteneur (1) sont répartis au moins deux orifices de chargement (6, 7, 8, 9, 11, 12, 13, 14) prévus pour le substrat, tout en notant que les orifices de chargement (6, 7, 8, 9, 11, 12, 13, 14) sont disposés dans la zone de chevauchement ou tangente des dispositifs de mélange voisins.

2. Dispositif de fermentation selon la revendication 1, **caractérisé en ce que** les orifices de chargement (6, 7, 8, 9, 11, 12, 13, 14) sont en relation via des conduites (5, 10) avec une alimentation commune pour le substrat.

3. Dispositif de fermentation selon la revendication 1 ou 2, **caractérisé en ce qu'**à chaque orifice de chargement (6, 7, 8, 9, 11, 12, 13, 14) soit attribué son dispositif de dosage.

4. Dispositif de fermentation selon la revendication 3, **caractérisé en ce que** les dispositifs de dosage sont en relation active avec une unité de commande centrale.

5. Dispositif de fermentation selon l'une des revendications 1 à 4, **caractérisé en ce que** dans la zone de transmission ou tangente des dispositifs de mélange (3) des éléments brisant le courant (16) sont prévus transversalement à l'allongement longitudinal du conteneur (1).

6. Procédé de décomposition biologique de substrat contenant de la matière organique et de récupération de biogaz se dégageant lors de la décomposition, tout en notant que le substrat est chargé dans un conteneur fermé et allongé (1), traverse dans le conteneur (1) plusieurs zones de réaction (2), dans lesquelles il y a circulation au moyen de dispositifs de mélange distincts (3) et les produits de décomposition provenant du conteneur (1) sont extraits, **caractérisés en ce que** le substrat soit chargé en au moins deux points sur l'allongement longitudinal du conteneur (6, 7, 8, 9, 11, 12, 13, 14) respectivement dans la zone de chevauchement ou tangente des dispositifs de mélange voisins dans le conteneur (1).

7. Procédé selon la revendication 6, **caractérisé en ce que** la quantité de substrat à charger dans le conteneur (1) est répartie et dosée dans le conteneur (1) en plus de la face avant du conteneur (1) aussi aux autres points discrets de chargement (6, 7, 8, 9, 11, 12, 13, 14) répartis sur la longueur du conteneur

8. Procédé selon la revendication 7, **caractérisé en ce que** les dosages soient commutés de façon cyclique ou successive.

9. Procédé selon la revendication 7 ou 8, **caractérisé en ce que** les dosages soient pilotés aux points de chargement (6, 7, 8, 9, 11, 12, 13, 14) de façon qu'un profil de valorisation équilibré soit obtenu sur tout le volume du conteneur.
